# EUROPÄISCHE PATENTSCHRIFT

(11) **EP 2 129 948 B1**
(45) Veröffentlichungstag und Bekanntmachung des Hinweises auf die Patenterteilung: **20.03.2013**
(21) Anmeldenummer: 08707258.3
(22) Anmeldetag: 24.01.2008
(51) Int. Cl.: F16K 11/076, F16K 11/085

(54) **MULTIFUNKTIONSVENTIL**
MULTIFUNCTION VALVE
SOUPAPE MULTIFONCTIONS

(30) Priorität: 25.01.2007 DE 102007003690
(43) Veröffentlichungstag der Anmeldung: 09.12.2009
(73) Patentinhaber: Edwards Lifesciences IPRM AG, 1260 Nyon (CH)
(72) Erfinder: MOULAS, Daniel, 83539 Pfaffing (DE); THOMAMÜLLER, Tobias, 83052 Bruckmühl (DE); FISCHER, Stefan, 83561 Ramerberg (DE); PFEIFFER, Ulrich, 81667 München (DE)
(74) Vertreter: Eisenführ, Speiser & Partner
(86) Internationale Anmeldenummer: PCT/EP2008/000547
(87) Internationale Veröffentlichungsnummer: WO 2008/089985

(56) Entgegenhaltungen:
- EP-A- 0 088 570
- FR-A- 675 640
- GB-A- 1 506 305
- US-A- 4 397 335
- US-B2- 6 918 893

## Beschreibung

Die Erfindung betrifft ein Multifunktionsventil mit mindestens drei Anschlüssen.

Im Stand der Technik ist aus der WO 2006/025054 A2 ein Absperrhahn mit einem Gehäuse, dass mindestens drei Anschlüsse bestimmt, bekannt. Auch die US 69 18893 B2 zeigt ein Multifunktions ventil mit den Merkmalen des Oberbegriffs von Anspruch 1.

Im Stand der Technik ist es bekannt, Druckaufnehmer an einer Halteplatte anzubringen, die sich etwa 150 cm vom Patienten entfernt befindet. Dadurch ist es erforderlich, eine mindestens ebenso lange Druckleitung vorzusehen. Durch Einflüsse dieser langen Druckleitung werden die Messergebnisse jedoch ungenau. Außerdem kann ein Berühren der Druckleitung die Messergebnisse verfälschen.

Es ist nicht möglich, mit Standardkomponenten einen Druckaufnehmer so zu verbinden, dass ein Drucksignal nahe am Katheterschlauch mit höherer Genauigkeit aufgenommen werden kann, ohne damit grundlegende Funktionen, wie beispielsweise Spülung und Blutentnahme eines solchen Messsystems zu beeinträchtigen.

Daraus ergibt sich die Aufgabe, eine Einrichtung bereitzustellen, mit der ein Drucksignal mit hoher Genauigkeit aufgenommen werden kann, die gleichzeitig die grundlegenden Funktionen, wie Spülung, Blutentnahme und Nullung des Druckaufnehmers bietet.

Diese Aufgabe wird gelöst durch ein Multifunktionsventil mit mindestens drei Anschlüssen, wobei ein erster Anschluss mit einem Katheter, ein zweiter Anschluss mit einer Infusion und ein dritter Anschluss mit einem Druckaufnehmer verbindbar ist, in einer ersten Stellung der erste und der zweite Anschluss über einen ersten Kanal miteinander verbunden sind, in einer zweiten Stellung der erste und der dritte Anschluss über einen zweiten Kanal miteinander verbunden sind und der zweite und der dritte Anschluss über einen dritten Kanal miteinander verbunden sind, in einer dritten Stellung der zweite und der dritte Anschluss über den zweiten Kanal miteinander verbunden sind und der erste Anschluss verschlossen ist und der dritte Kanal einen kleineren Querschnitt als der zweite Kanal aufweist. Dabei ist vorteilhaft, dass der Druckaufnehmer geschützt ist, wenn ein Draht durch den zweiten und ersten Anschluss geschoben wird. Der Draht wird in dem ersten Kanal geführt, der in der ersten Stellung keine Verbindung zu dem Druckaufnehmer hat, so dass ein Kontakt zwischen Draht und Druckaufnehmer verhindert wird. Auch das Einbringen von Gel aus einer Gel-Übertragungsmembran eines Drucksensors mit dem Draht in den Katheter wird so verhindert.

Als Multifunktionsventil wird vorzugsweise ein Mehrwegehahn eingesetzt, der die Strömung zwischen drei Anschlüssen steuert. Besonders bevorzugt wird ein Absperrhahn eingesetzt, der einen oder mehrere der mit ihm verbundenen Wege absperren kann.

Die Anschlüsse sind vorzugsweise biokompatibel. Vorzugsweise sind die Anschlüsse auch flüssigkeitsdicht, so dass sie mit Kanülen, Spritzen, Infusionsschläuchen, Kathetern und/oder Druckaufnehmern verbindbar sind. Vorzugsweise sind die Anschlüsse zur Sicherung bzw. Verrieglung der Verbindung gegen versehentliches Lösen mit einem Gewinde und einer Überwurfmutter versehen. Die Anschlüsse sind vorzugsweise zylindrisch, besonders bevorzugt kegelförmig. Vorzugsweise sind die Anschlüsse an dem Multifunktionsventil Innenkegel bzw. Innenzylinder. In einer anderen bevorzugten Ausführungsform sind die Anschlüsse an dem Multifunktionsventil Außenkegel, bzw. Außenzylinder. Der Anschluss wird vorzugsweise durch Aufeinanderzuschieben mit den anzuschließenden Bauteilen verbunden. Vorzugsweise sind die Anschlüsse des Multifunktionsventils stumpf vorgesehen und für eine Klebeverbindung ausgeführt. Ebenfalls bevorzugt ist eine Verbindung, die durch Quetschen oder Klemmen hergestellt wird. Besonders bevorzugt wird diese Verbindung durch eine Drehung höchst bevorzugt durch eine halbe Drehung geschlossen und geöffnet. Die Anschlüsse sind besonders bevorzugt als Luer-Lock-Anschlüsse ausgebildet.

Als Katheter können alle Katheter eingesetzt werden, die ein offenes Lumen besitzen. Vorzugsweise werden-Herzkatheter oder Venenkatheter, besonders bevorzugt arterielle Katheter eingesetzt. Als Katheter werden Röhrchen oder Schläuche, mit denen Hohlorgane sondiert, entleert, gefüllt und/oder gespült werden können oder der Druck in diesen gemessen werden kann, eingesetzt.

Die Flüssigkeiten der Infusion werden vorzugsweise intravenös oder intraarteriell verabreicht. Als Flüssigkeiten werden vorzugsweise Spüllösungen, Blut, Blutersatzstoffe, Plasmaersatzstoffe, Elektrolytlösungen, nichtionische Lösungen und/oder Medikamente eingesetzt. Besonders bevorzugt werden Glukoselösung, Kochsalzlösung und Ringer-Laktat Lösung eingesetzt.

Der Druckaufnehmer misst vorzugsweise den absoluten Druck, besonders bevorzugt den Differenzdruck. Die Größe des Druckaufnehmers liegt vorzugsweise zwischen 1/10 mm und 10 cm.

Der erste Kanal ist vorzugsweise eine Vertiefung in dem Multifunktionsventil besonders bevorzugt ein Durchgangsloch. Der zweite Kanal ist vorzugsweise ein Durchgangsloch, besonders bevorzugt eine Vertiefung in dem Multifunktionsventil. Der dritte Kanal ist vorzugsweise eine Vertiefung in dem Multifunktionsventil besonders bevorzugt ein Durchgangsloch.

In der zweiten Stellung kann über den dritten Anschluss der Druck des durch den zweiten Kanal zugeführten Gases und/oder der Flüssigkeit gemessen werden, während das Multifunktionsventil gleichzeitig über den dritten Kanal gespült wird. Da der dritte Kanal einen kleineren Querschnitt als der zweite Kanal aufweist, sind die Einflüsse des Spülens auf den Druck minimal.

Die Querschnittsfläche der Kanäle entspricht dem Flächeninhalt, der bei einem Schnitt im Winkel von 90° zur Längsachse des Kanals freigelegten Fläche.

Vorzugweise liegen der erste und der zweite Anschluss einander gegenüber. Dadurch können Gase und/oder Flüssigkeiten ohne Änderungen der Flussrichtung von dem ersten in den zweiten Anschluss oder umgekehrt gelangen. Energieverluste der hindurchströmenden Gase und/oder Flüssigkeiten durch Reibung und/oder Turbulenzen werden so minimiert.

Der erste und der zweite Anschluss sind vorzugsweise zu gegenüberliegenden Seiten einer Ebene hin geöffnet, die zwischen dem ersten und dem zweiten Anschluss verläuft. Besonders bevorzugt ist der Winkel zwischen dem ersten und dem zweiten Anschluss größer als 90°. Besonders bevorzugt beträgt der Winkel zwischen dem ersten und dem zweiten Anschluss 180°.

Das Multifunktionsventil weist bevorzugt ein Gehäuse auf. Das Gehäuse schützt das Innere des Multifunktionsventils. Es schließt das Innere des Multifunktionsventils von der Umgebung ab. Vorzugsweise ist das Gehäuse flüssigkeitsdicht, so dass Flüssigkeiten aus dem Inneren nicht in die Umgebung gelangen können.

Auf dem Gehäuse des Multifunktionsventils sind vorzugsweise Markierungen für eine oder mehrere Schaltstellungen vorgesehen. Vorzugsweise weist das Gehäuse auch Verrastungen für eine oder mehrere Schaltstellungen auf. Besonders bevorzugt weist das Gehäuse Anschläge für eine oder mehrere Schaltstellungen auf.

Bevorzugt ist der Druckaufnehmer mit dem Gehäuse verbunden, besonders bevorzugt ist der Druckaufnehmer unmittelbar in oder an dem Gehäuse angebracht. Dadurch kann der Druck unmittelbar in oder an dem Multifunktionsventil gemessen werden. Auf diese Weise ist eine zeitlich hoch auflösende Messung erzielbar. Diese Messung ist daher für die Analyse der naturgetreuen Form einer Druckkurve besonders geeignet. Außerdem werden negative Einflüsse zusätzlicher Leitungen so beseitigt.

Besonders bevorzugt ist der dritte Kanal eine Kapillare. Dadurch ist der Einfluss des Spülens auf das Ergebnis der Druckmessung besonders gering.

Die Kapillare ist vorzugsweise ein sehr feiner lang gestreckter Hohlraum. Besonders bevorzugt ist die Kapillare als Glaskapillare oder gelasertes Loch ausgeführt.

Vorzugsweise liegt die Länge der Kapillare zwischen 1/10 mm und 12 mm und der Durchmesser zwischen 1/100 mm und 4/10 mm. Dadurch sind Durchflussraten zwischen 2 und 20 ml pro Stunde bei Beaufschlagung der Spüllösung mit üblichen Drucken von ca. 300 mmHg erreichbar.

Der erste Kanal, der zweite Kanal und der dritte Kanal sind vorzugsweise in einer Schalteinrichtung angeordnet, die in dem Gehäuse drehbar ist. Dadurch wird eine Verbindung der Anschlüsse durch die Kanäle entsprechend den unterschiedlichen Stellungen auf einfache Weise realisiert. Der dritte Kanal ist besonders bevorzugt in dem Gehäuse bzw. zum Teil in dem Gehäuse und zum Teil in der Schalteinrichtung vorgesehen. Wenn der dritte Kanal teilweise oder vollständig in dem Gehäuse vorgesehen ist, ist er vorzugsweise als Kapillare ausgebildet.

Die Schalteinrichtung ist vorzugsweise dazu eingerichtet, die Richtung oder Stärke von Durchflüssen zu beeinflussen. Besonders bevorzugt wird dies durch ein mechanisches Bauteil erreicht, das durch eine Lage oder Positionsänderung die Strömung beeinflussen kann. Besonders bevorzugt ist die Schalteinrichtung in dem Gehäuse drehbar. Vorzugsweise ist die Schalteinrichtung um eine ihrer eigenen Achsen drehbar. Besonders bevorzugt ist die Schalteinrichtung zweiteilig, da sie so besonders einfach herstellbar ist.

Der erste Kanal weist vorzugsweise leichte Knicke mit einem Winkel von unter 6° auf. Besonders bevorzugt ist der erste Kanal als Durchgangsloch ausgebildet. Dadurch können in der ersten Stellung der erste und der zweite Anschluss auf einfache Weise verbunden werden. Ausserdem wird dadurch auch die Anwendung eines Führungsdrahtes vereinfacht. Bei Knicken mit Winkeln von über 6° könnte der Führungsdraht Schäden erleiden oder Reibung im Lumen hervorrufen. Außerdem werden Richtungsänderungen eines hindurchströmenden Gases oder einer Flüssigkeit in dem ersten Kanal vermieden.

Das Durchgangsloch weist vorzugsweise an den beiden entgegen gesetzten Enden seiner Längsachse Öffnungen auf. Besonders bevorzugt sind die Öffnungen beide so groß wie der Querschnitt des Durchgangslochs. Besonders bevorzugt ist die Größe des Querschnitts des Durchgangslochs in allen Bereichen des Durchgangslochs gleich.

Vorzugsweise verläuft der erste Kanal quer durch die Schalteinrichtung. So können der erste und der zweite Anschluss auf besonders einfache Weise miteinander verbunden werden.

Der erste Kanal verläuft vorzugsweise nicht parallel zur Drehachse der Schalteinrichtung. Besonders bevorzugt verläuft der erste Kanal in einer Richtung, die senkrecht auf der Drehachse der Schalteinrichtung steht. Besonders bevorzugt schneidet der erste Kanal die Drehachse der Schalteinrichtung.

Ein Richtungsvektor des ersten Kanals, ein Richtungsvektor des dritten Kanals und ein Vektor, der von einem Aufpunkt des ersten Kanals zu einem Aufpunkt des dritten Kanals zeigt, sind vorzugsweise linear unabhängig. Dabei ist ein Richtungsvektor des ersten Kanals ein Vektor, der in der ersten Stellung des Multifunktionsventils in Fließrichtung eines Fluids von dem ersten Anschluss zu dem zweiten Anschluss zeigt. Ein Richtungsvektor des dritten Kanals ist ein Vektor, der in der zweiten Stellung des Multifunktionsventils in Fließrichtung eines Fluids von dem dritten Anschluss zu dem zweiten Anschluss zeigt.

Linear unabhängig bedeutet vorzugsweise, dass sich keiner der Vektoren als Linearkombination der anderen Vektoren darstellen lässt.

Bevorzugt sind der erste Kanal und der dritte Kanal nicht mit einander verbunden. Dadurch wird eine Befüllung des ersten Kanals durch den dritten Kanal in der zweiten und der dritten Stellung vermieden.

Zwischen dem ersten und dem dritten Kanal besteht vorzugsweise keine Durchgangsmöglichkeit für Flüssigkeiten, besonders bevorzugt keine Durchgangsmöglichkeit für Gase.

Vorzugsweise ist der zweite Kanal als Rille in der Schalteinrichtung ausgebildet. Dadurch kann der zweite Kanal auf besonders einfache Weise gefertigt werden.

Die Rille ist vorzugsweise eine Vertiefung in der Schalteinrichtung. Besonders bevorzugt ist die Rille eine lang gestreckte Vertiefung.

Der zweite Kanal verläuft bevorzugt im Wesentlichen in Umfangsrichtung der Schalteinrichtung. Dadurch ist es auf einfache Weise möglich, Anschlüsse, die in Umfangsrichtung angeordnet sind, miteinander zu verbinden.

Vorzugsweise erstreckt sich der zweite Kanal mit mehr als 50 % seiner Länge in Umfangsrichtung der Schalteinrichtung. Besonders bevorzugt erstreckt er sich mit mehr als 70 % seiner Länge in Anfangsrichtung der Schalteinrichtung.

Der zweite und der dritte Kanal sind vorzugsweise miteinander verbunden. Dadurch ist es möglich, drei Anschlüsse miteinander zu verbinden.

Der zweite und dritte Kanal sind vorzugsweise mit einem Durchlass für Gase, besonders bevorzugt mit einem Durchlass für Flüssigkeiten verbunden.

Bevorzugt weist der zweite Kanal in einem Endbereich eine Aufweitung auf und ist im Bereich der Aufweitung mit dem dritten Kanal verbunden. Dadurch ist es besonders einfach, den dritten Kanal an dem ersten Kanal vorbei zu führen.

Vorzugsweise erstreckt sich die Aufweitung des zweiten Kanals in dem Endbereich in Längsrichtung der Schalteinrichtung. In dem Bereich des Kanals, der sich in Längsrichtung der Schalteinrichtung erstreckt, endet der dritte Kanal vorzugsweise.

Der zweite Kanal ist vorzugsweise als Durchgangsloch ausgebildet. Dadurch sind die benetzten Dichtflächen zwischen dem Gehäuse und der Schalteinrichtung bei einer Druckmessung klein. Dadurch kann die Entstehung von Ablagerungen besonders gut verhindert werden.

Der Querschnitt des zweiten Kanals ist vorzugsweise über die gesamte Länge des Kanals gleich. Besonders bevorzugt weist der Kanal einen runden Querschnitt auf. Dadurch werden die Eigenschaften einer Strömung eines durch den Kanal fließenden Fluids in besonders geringem Maß geändert.

Vorzugsweise weist ein Kathetersystem ein Multifunktionsventil auf, das mit einem Katheterschlauch verbindbar ist. Dadurch wird ein Kathetersystem bereitgestellt, das durch den Katheterschlauch mit dem Körper eines Patienten verbindbar ist und durch das Multifunktionsventil mit medizinischen Einrichtungen verbindbar ist.

Ein Kathetersystem ist ein System, das vorzugsweise mehrere Bauteile aufweist, wobei eines der Bauteile ein Katheterschlauch ist.

Ein Katheterschlauch ist ein Schlauch aus Metall, Glas vorzugsweise Kunststoff, besonders bevorzugt aus Silikon, Polyethylen oder Polyurethan, mit dem Hohlorgane sondiert, entleert, gefüllt oder gespült werden können oder der Druck darin gemessen werden kann. Vorzugsweise ist der Schlauch verformbar.

Das Kathetersystem weist vorzugsweise ein Multifunktionsventil auf, an dem ein Druckaufnehmer anschließbar ist. Dadurch kann der Druck in dem Katheterschlauch gemessen werden. Der gemessenen Druckwerte sind besonders genau, da ganz nahe am interessierenden Kreislaufabschnitt des Patienten gemessen wird.

Das Kathetersystem weist vorzugsweise einen Druckaufnehmer auf, an den eine Steckverbindung anschließbar ist. Dadurch können an den Druckaufnehmer Einrichtungen angeschlossen werden. Bevorzugt sind diese Einrichtungen Warneinrichtungen, die beim Überschreiten und/oder Unterschreiten eines kritischen Drucks ein optisches, vorzugsweise akustisches Warnsignal erzeugen. Diese Einrichtungen sind besonders bevorzugt Aufzeichnungseinrichtungen zum Aufzeichnen des Drucks. Der Druck wird vorzugsweise als Ausdruck, besonders bevorzugt elektronisch, analog, digital oder numerisch und besonders bevorzugt in Kurvenform aufgezeichnet.

Eine Steckverbindung ist eine Verbindung, durch die Elektrizität, vorzugsweise optische Signale, besonders bevorzugt elektrische Signale aus einer Baugruppe in eine andere Baugruppe übertragbar sind.

Vorzugsweise weist eine Vorrichtung ein Kathetersystem auf, das mit einem Blutentnahmeport verbindbar ist. Dadurch kann einem Patienten auf besonders einfache Weise Blut entnommen werden. Vorzugsweise wird das Kathetersystem dabei nicht gegen die Atmosphäre geöffnet. So wird Kontamination des Patientenblutes durch Bakterien und Viren vermieden.

Ein Blutentnahmeport ist eine verschließbare Öffnung zum Entnehmen von Blut. Der Blutentnahmeport ist als Schraubverbindung mit Deckel, vorzugsweise als Steckverbindung mit Deckel, besonders bevorzugt als durchstechbares Bauteil ausgeführt. Das durchstechbare Material ist aus einem zähen Material wie Kunststoff, vorzugsweise Gel ausgestaltet. Das durchstechbare Bauteil kann mit einer Nadel durchstochen werden und schließt sich vorzugsweise wieder, sobald die Nadel herausgezogen wird.

Die Vorrichtung weist vorzugsweise ein Kathetersystem auf, das mit einem Reservoir mit beweglichem Kolben verbindbar ist. Dadurch können einem Patienten auf besonders einfache Weise Medikamente zugeführt werden.

Das Reservoir mit beweglichem Kolben ist ein Hohlraum zur Aufnahme von Stoffen. Vorzugsweise sind die Stoffe durch den beweglichen Kolben aus dem Hohlraum heraus bewegbar. Vorzugsweise ist das Reservoir mit beweglichem Kolben eine Spritze. Vorzugsweise ist die Spritze an ihrem Ende mit einem Gewinde versehen. Dadurch kann die Spritze ausgetauscht werden. Die Spritze weist vorzugsweise Glaskomponenten auf, besonders bevorzugt besteht die Spritze aus Kunststoff.

Die Vorrichtung weist vorzugsweise ein Kathetersystem auf, das mit einer Abgleichvorrichtung verbindbar ist. Dadurch kann auf einfache Weise ein Druckabgleich an dem Druckaufnehmer erzeugt werden.

Die Abgleichvorrichtung ist vorzugsweise ein Umlenkhahn mit mindestens zwei Anschlüssen. Einer der Anschlüsse ist vorzugsweise mit dem Kathetersystem verbunden. Der zweite Anschluss ist vorzugsweise zur Atmosphäre offen. Der Umlenkhahn ist vorzugsweise in verschiedene Stellungen bringbar, wobei vorzugsweise in mindestens einer der Stellungen das Kathetersystem mit der Atmosphäre verbunden ist. Besonders bevorzugt weist die Abgleichvorrichtung einen dritten Anschluss auf, mit dem vorzugsweise ein Sicherheitsventil verbindbar ist. In der Stellung des Umlenkhahns, in der das Kathetersystem mit der Atmosphäre verbunden ist, wird der Druckaufnehmer dem atmosphärischen Druck ausgesetzt, so dass er kalibriert werden kann. Es gibt spezielle Drucksensoren, die ab Werk eine so hohe Genauigkeit aufweisen, dass eine Kalibrierung mit einer speziellen Abgleichvorrichtung nicht erforderlich ist.

Die Vorrichtung weist vorzugsweise ein Kathetersystem auf, das mit einem Sicherheitsventil verbindbar ist. Dadurch kann die Vorrichtung auf einfache Weise geschlossen werden.

Das Sicherheitsventil ist ein Ventil, mit dem ein Fluss in der Vorrichtung begrenzbar ist, insbesondere im Fall der Injektion kann hierdurch ein Rückfluss in den Spülbeutel verhindert werden.

Das Sicherheitsventil ist vorzugsweise mit einer Spülkapillare versehen. Dadurch wird eine kontinuierliche Spülung der Vorrichtung insbesondere mit Fluiden wie beispielsweise NaCl-Lösung sichergestellt und eine Flüssigkeitsgabe ermöglicht.

Die Vorrichtung weist vorzugsweise ein Kathetersystem auf, das mit einer Tropfkammer verbindbar ist.

Die Tropfkammer ist eine Einrichtung zur Leitung von Flüssigkeiten, die vorzugsweise eine Unterbrechung des Flüssigkeitsfilms bewirkt. Die Tropfkammer ist ein Behältnis mit vorzugsweise zwei Anschlüssen. An den ersten Anschluss ist vorzugsweise ein Tropfenerzeuger angeschlossen. Die Flüssigkeit wird vorzugsweise tropfenweise von dem Tropfenerzeuger in die Tropfkammer eingeleitet. Vorzugsweise wird die Flüssigkeit in der Tropfkammer gesammelt. Vorzugsweise wird die Flüssigkeit aus der Tropfkammer wieder abgegeben.

Die Vorrichtung weist vorzugsweise ein Kathetersystem auf, das mit einem Spülbeutel verbindbar ist. Dadurch kann die Vorrichtung auf einfache Weise mit einer Spüllösungseinrichtung gespült werden.

Die Spüllösungseinrichtung ist vorzugsweise eine Spritzenpumpe, besonders bevorzugt ein mit Druck beaufschlagter Spülbeutel oder eine Flasche.

Der Spülbeutel ist ein Beutel, der vorzugsweise zur Aufnahme einer Spülflüssigkeit eingerichtet ist. Vorzugsweise besteht der Spülbeutel aus Kunststoff, besonders bevorzugt aus durchsichtigem Kunststoff. Dadurch ist der Flüssigkeitsstand sichtbar. Der Spülbeutel weist vorzugsweise mindestens einen Anschluss zum Verbinden mit anderen Bauteilen auf. Vorzugsweise ist der Spülbeutel verformbar und wird durch einen umgebenden Hohlbeutel pneumatisch mit Druck beaufschlagt. Dadurch kann Flüssigkeit aus dem Spülbeutel abgegeben werden, ohne dass ein anderes Fluid hineingegeben werden muss.

Die Erfindung wird im Folgenden beispielhaft anhand der beigelegten Figuren beschrieben. Es zeigen:
- Fig. 1: einen Querschnitt eines erfindungsgemäßen Dreiwegehahns in einer ersten Stellung;
- Fig. 1a: eine Seitenansicht der Schalteinrichtung des in Fig. 1 gezeigten Dreiwegehahns;
- Fig. 2: einen Querschnitt des erfindungsgemäßen Dreiwegehahns aus Fig. 1 in einer zweiten Stellung;
- Fig. 3: einen Querschnitt des erfindungsgemäßen Dreiwegehahns aus den Fig. 1 und 2 in einer dritten Stellung;
- Fig. 4: einen Querschnitt durch eine zweite Ausführungsform eines erfindungsgemäßen Dreiweghahns;
- Fig. 4a: eine Seitenansicht der Schalteinrichtung des in Fig. 4 gezeigten Dreiwegehahns;
- Fig. 5a: einen Querschnitt durch eine dritte Ausführungsform eines erfindungsgemäßen Dreiwegehahns in der ersten Stellung;
- Fig. 5b: einen Querschnitt durch die dritte Ausführungsform des erfindungsgemäßen Dreiwegehahns in der zweiten Stellung; und
- Fig. 5c: einen Querschnitt durch die dritte Ausführungsform des erfindungsgemäßen Dreiwegehahns in der dritten Stellung;
- Fig. 5d: eine Seitenansicht der Schalteinrichtung des in Fig. 5a, 5b und 5c gezeigten Dreiwegehahns;
- Fig. 6: eine schematische Ansicht eines Monitoring-Katheter-Sets;
- Fig. 7a-c: eine schematische Darstellung einer vierten Ausführungsform des Dreiwegehahns;
- Fig. 8a-c: eine schematische Darstellung einer fünften Ausführungsform des Dreiwegehahns;
- Fig. 9: einen Querschnitt durch eine schematische Darstellung einer sechsten Ausführungsform des Dreiwegehahns;
- Fig. 10a-b: Ansichten eines Hebels;
- Fig. 11a-b: Ansichten eines zweiten Hebels;
- Fig. 12a-d: Ansichten einer Schiebemechanik;
- Fig. 13a-d: Ansichten einer Mechanik zum Verdrehen der Gehäusehälften gegeneinander und
- Fig. 14a-f: Ansichten eines Wippschalters.

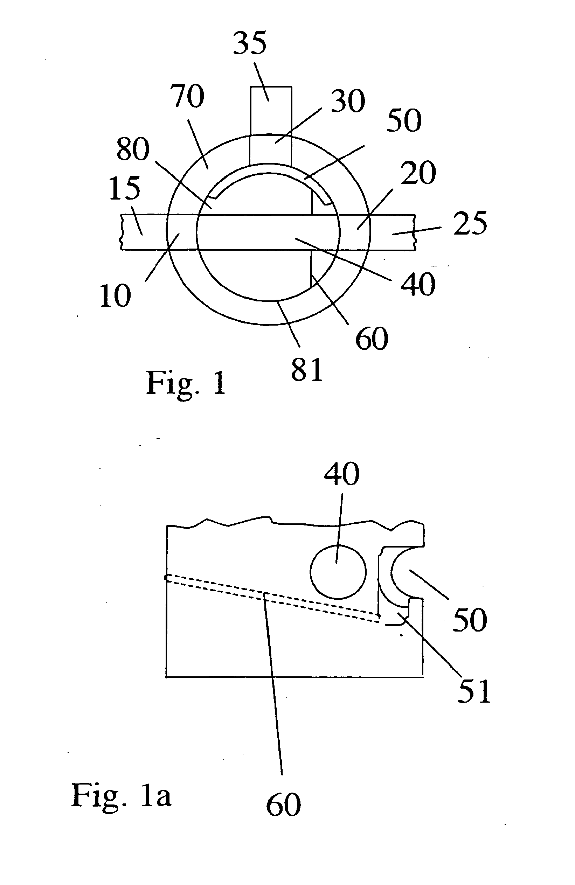

Fig. 1 zeigt einen Dreiwegehahn 1 in einer ersten Stellung. Der Dreiweghahn 1 weist ein im Querschnitt kreisringförmiges Gehäuse 70 auf, das aus Kunststoff besteht. Der Durchmesser des Gehäuses 70 beträgt 6 mm Das Gehäuse 70 hat drei Anschlüsse 10, 20, 30. Die drei Anschlüsse 10, 20, 30 liegen in der gleichen Querschnittsebene. Der erste und der zweite Anschluss 10, 20 liegen einander gegenüber. Der dritte Anschluss 30 liegt zu den beiden anderen Anschlüssen um 90° verdreht auf dem Querschnitt des Gehäuses.

Der erste Anschluss ist zur Verbindung mit einem Katheter 15 vorgesehen. Der Katheter 15 ist hier ein arterieller Katheter 15, der mit der Seldingertechnik in einer Arterie eines Patienten eingebracht werden kann.

Dabei wird die Arterie an der entsprechenden Stelle (z. B. am Hals, Bein oder am Arm) mit einer Art Verweilkanüle punktiert. Nach Entfernen der Kanüle wird über den nun im Blutgefäß liegenden Plastikschlauch der eigentliche Draht vorgeschoben. Der Schlauch wird dann so entfernt, dass der Führungsdraht weiterhin intravasal liegt. Nun wird der arterielle Katheter über den Draht in das Blutgefäß vorgeschoben. Anschließend wird der Führungsdraht entfernt und der Katheter durchgespült.

Der zweite Anschluss 20 ist mit einer Infusion 25 verbunden. Als Infusion 25 ist hier eine Kochsalzlösung vorgesehen.

Der dritte Anschluss 30 ist mit einem Druckaufnehmer 35 verbunden.

Im Inneren des Gehäuses 70 befindet sich eine Schalteinrichtung 80. Der Querschnitt der Schalteinrichtung 80 ist kreisförmig. Die Schalteinrichtung 80 liegt auf der Innenseite des Gehäuses 70 an. Sie weist einen ersten Kanal 40 auf, der als Durchgangsloch ausgebildet ist, und sich quer durch die Schalteinrichtung 80 erstreckt. In der in Fig. 1 gezeigten Stellung verbindet der erste Kanal 40 den ersten Anschluss 10 mit dem zweiten Anschluss 20. Der Querschnitt des Kanals 40 ist an allen Stellen gleich und entspricht dem Querschnitt des ersten und zweiten Anschlusses 10, 20.

Der zweite Kanal 50 ist als Vertiefung in der Schalteinrichtung 80 ausgebildet. Der zweite Kanal 50 erstreckt sich in Umfangsrichtung auf der Schalteinrichtung 80 und weist wie in Fig. 1a erkennbar an einem Ende eine Aufweitung 51 auf. In der in Fig. 1 gezeigten Stellung liegt der zweite Kanal 50 an dem dritten Anschluss 30 an. Der dritte Kanal 60 ist als gelaserte Kapillare ausgeführt. Er erstreckt sich von der Aufweitung des zweiten Kanals 50 aus an dem ersten Kanal 40 vorbei zur Außenfläche 81 der Schalteinrichtung 80.

In der in der Fig. 1 gezeigten Stellung ist der Katheter 15 mit der Infusion 25 verbunden. Der dritte Anschluss 30 zu dem Druckaufnehmer 35 ist verschlossen.

In dieser Stellung wird eine Schnellspülung des Dreiwegehahns 1 mit Kochsalzlösung durchgeführt. In dieser Stellung könnte auch auf der Infusionsseite eine Blutprobe entnommen werden. Dazu ist an der Infusionsleitung eine Blutentnahmestelle vorgesehen, die mit 30 ml/h gespült wird. Außerdem kann in dieser Stellung der Führungsdraht zum Anlegen des arteriellen Katheters mit der Seldingertechnik durchgeführt werden.

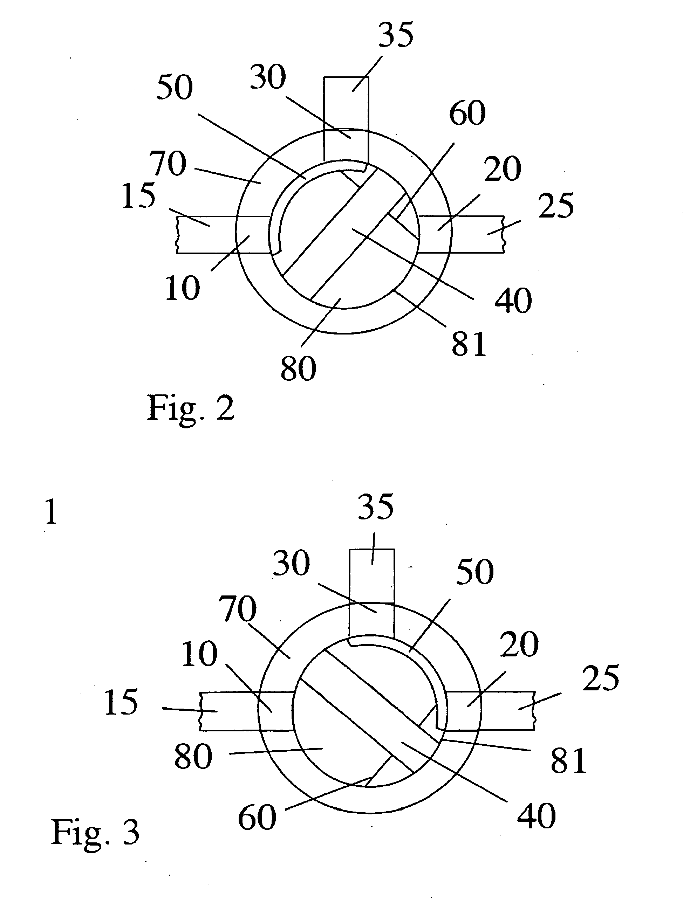

Fig. 2 zeigt den Dreiwegehahn 1 aus Fig. 1 in einer zweiten Stellung. Die Schalteinrichtung 80 ist im Vergleich zu der Stellung in Fig. 1 um 45° gegen den Uhrzeigersinn um die eigene Achse gedreht. Der erste Anschluss 10 und der dritte Anschluss 30 sind durch einen zweiten Kanal 50 verbunden. Der dritte Kanal 60 verbindet den dritten Anschluss 30 mit dem zweiten Anschluss 20.

In dieser Stellung wird eine Pulskonturanalyse durchgeführt. Die Pulskonturanalyse ist ein Verfahren zur Bestimmung des Herzminutenvolumens. Dabei wird der arterielle Blutdruck über der Zeit bestimmt und daraus das Schlagvolumen des Herzen berechnet. Eine valide Durchführung dieser Methode erfordert eine gute Qualität der abgeleiteten Druckkurve. In der in Fig. 2 gezeigten Stellung ist der Druckaufnehmer mit dem Katheter 15 verbunden. In dem Katheter 15 liegt ein mittlerer Druck von 100 mm Hg an. Durch den dritten Kanal 60 fließt eine Kochsalzlösung von der Infusion 25 zu dem Druckaufnehmer 35. In der Infusion 25 liegt ein Druck von 300 mm Hg an, so dass die Kochsalzlösung durch den Dreiwegehahn 1 in das Blut fließen kann. Dadurch, dass die Kochsalzlösung den Dreiwegehahn 1 bei der naturgetreuen Druckmessung kontinuierlich spült, wird das Entstehen von Ablagerungen verhindert.

Der Querschnitt des dritten Kanals 60 ist so dünn, dass weniger als 5 ml Kochsalzlösung pro Stunde hindurch fließen. Diese Menge ist so gering, dass die Druckmessung davon nicht beeinflusst wird.

Da der Druckaufnehmer 35 direkt an dem Dreiwegehahn 1 sitzt, werden bei der Druckmessung Leitungseinflüsse vermieden. Auf diese Weise werden die Drücke sehr genau gemessen.

In Fig. 3 ist der Dreiwegehahn 1 in einer dritten Stellung gezeigt. In dieser Stellung ist die Schalteinrichtung 80 im Vergleich zu der in Fig. 1 gezeigten Stellung um 45° mit dem Uhrzeigersinn gedreht. Der zweite Kanal 50 verbindet den dritten Anschluss 30 mit dem zweiten Anschluss 20. Der erste Anschluss 10 ist verschlossen.

In dieser Stellung wird der Nullabgleich des Druckaufnehmers vorgenommen, indem dieser zur Atmosphäre geöffnet wird.

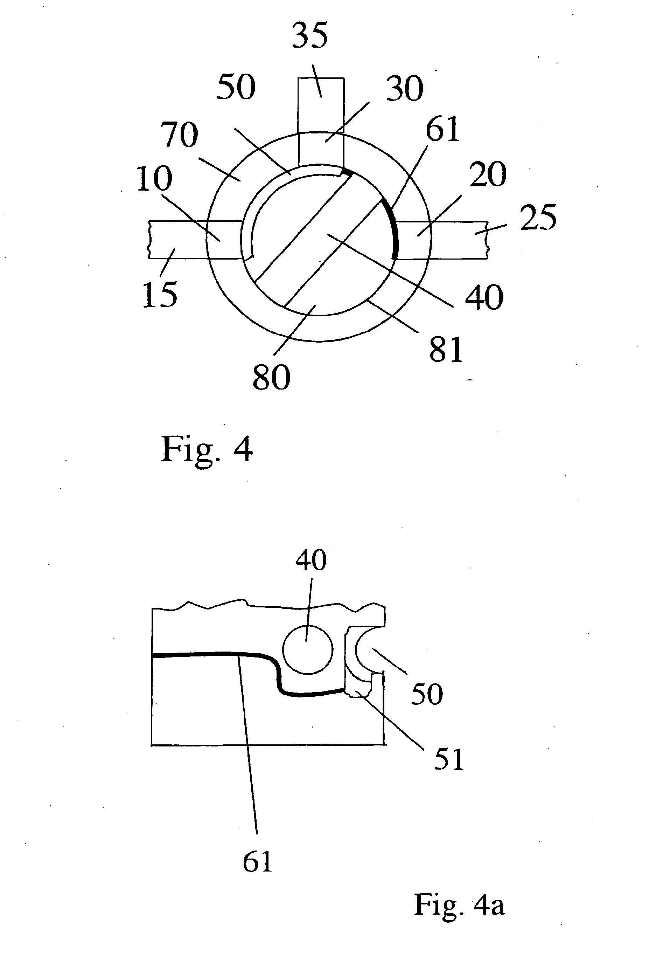

In den Fig. 4 und 4a ist eine zweite Ausführungsform des erfindungsgemäßen Dreiwegehahns 1 gezeigt. Ein dritter Kanal 61 verläuft hier auf der Außenfläche 81 der Schalteinrichtung 80. Er ist als Rille ausgebildet. Wie in Fig. 4a zu sehen, verläuft er von der Aufweitung des zweiten Kanals 50 aus, um den ersten Kanal 40 herum und dann in Umfangsrichtung der Schalteinrichtung 80.

Der dritte Kanal 61 weist hier einen besonders kleinen Querschnitt auf. Dadurch, dass der kleine Kanal auf der Außenfläche 81 der Schalteinrichtung 80 vorgesehen ist, ist es trotzdem auf einfache Weise möglich, die Schalteinrichtung 80 als Kunststoffgussteil zu fertigen.

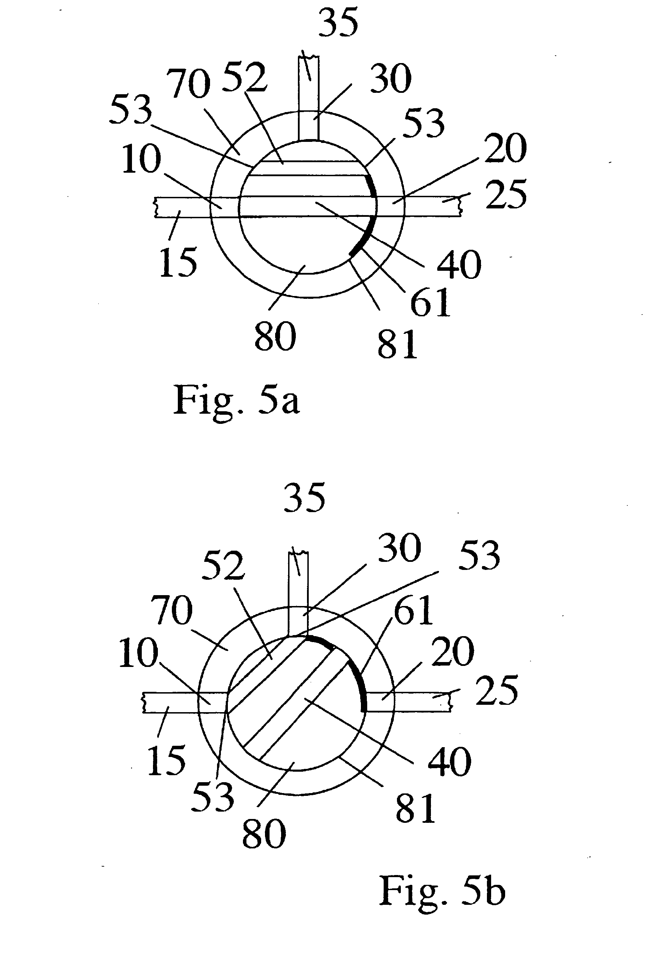

Fig. 5a zeigt einen Querschnitt durch eine dritte Ausführungsform eines erfindungsgemäßen Dreiwegehahns 1 in der ersten Stellung. Abweichend zu der in Figur 4 und 4a dargestellten Ausführungsform ist hier der zweite Kanal 52 nicht als Vertiefung in der Schalteinrichtung 80, sondern als Durchgangskanal ausgeführt. Der zweite Kanal 52 verläuft parallel zu dem ersten Kanal 40. Wie in den vorherigen Ausführungsformen ist der zweite Kanal 52 mit der Kapillare 61 verbunden.

In der ersten Stellung liegen die beiden Öffnungen 53 des zweiten Kanals 52 innen an dem Gehäuse 70 an. Der zweite Kanal 52 ist verschlossen.

Fig. 5b zeigt einen Querschnitt durch die dritte Ausführungsform des erfindungsgemäßen Dreiwegehahns 1 in der zweiten Stellung. Die Schalteinrichtung 80 ist hier um 45° gegen den Uhrzeigersinn gedreht, so dass der zweite Kanal 52 den ersten Anschluss 10 mit dem dritten Anschluss 30 verbindet.

Wie bei den oben beschriebenen Ausführungsformen kann in dieser Stellung der Druck gemessen werden. Es ist möglich, eine kontinuierliche Spülung des zweiten Kanals 52 durch die Kapillare 61 vorzunehmen. Die durch die Kapillare 61 geleitete Durchflussmenge ist 3 ml/h.

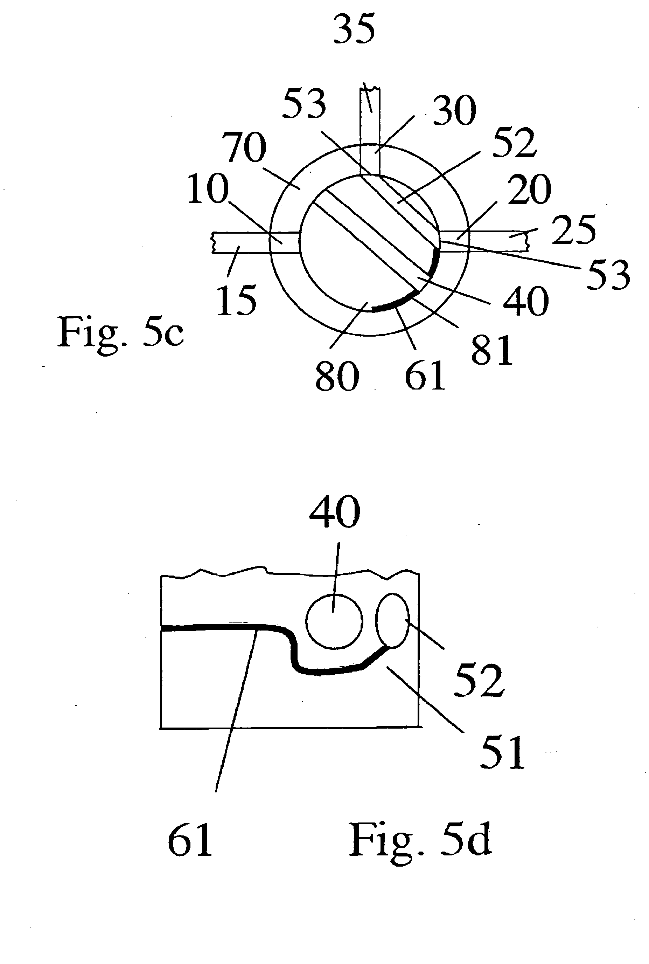

Fig. 5c zeigt einen Querschnitt durch die dritte Ausführungsform des erfindungsgemäßen Dreiwegehahns 1 in der dritten Stellung. Die Schalteinrichtung 80 ist hier um 45° mit dem Uhrzeigersinn gedreht, so dass der zweite Kanal 52 den dritten Anschluss 30 mit dem zweiten Anschluss 20 verbindet.

Wie in den oben beschriebenen Ausführungsformen ist es in dieser Stellung möglich, einen Nullabgleich des Druckaufnehmers 35 durchzuführen.

Dadurch, dass der zweite Kanal 52 hier als Durchgangskanal ausgeführt ist, sind die Dichtbereiche des zweiten Kanals 52 zwischen der Schalteinrichtung 80 und dem Gehäuse 70 verkleinert worden. Das Vermeiden von Ablagerungen in den Dichtbereichen des Kanals 50 ist so noch einfacher möglich.

Figur 5d zeigt eine Seitenansicht der Schalteinrichtung des in den Figuren 5a, 5b und 5c gezeigten Dreiwegehahns. Hier ist gezeigt, wie der dritte Kanal (61) von dem zweiten Kanal (52) aus um den ersten Kanal (40) herumgeführt ist.

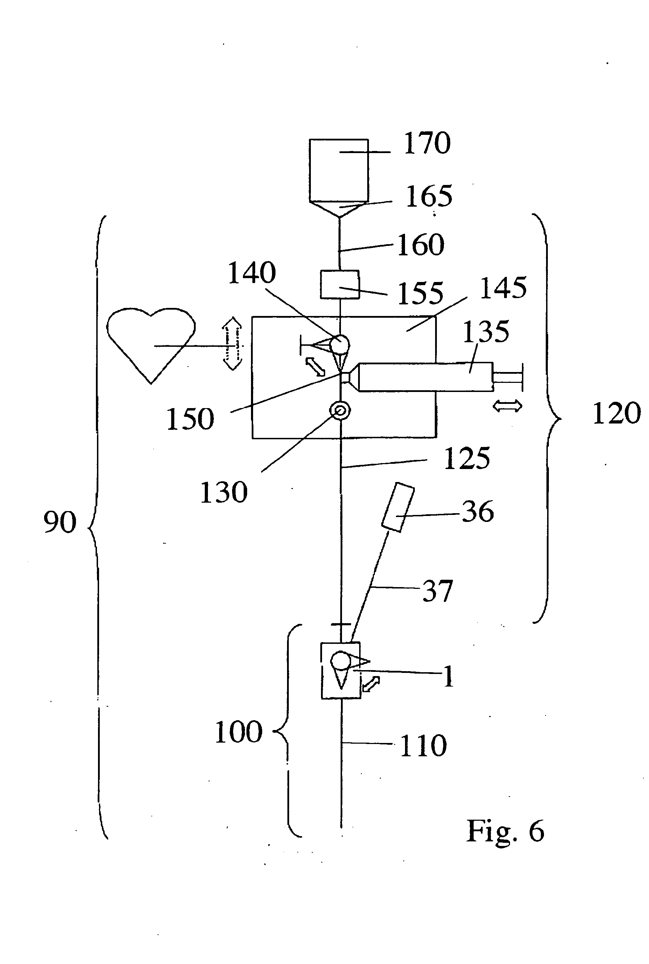

Fig. 6 zeigt eine schematische Ansicht eines Monitoring-Katheter-Sets 90. Das Monitoring-Katheter-Set 90 weist einen Sensor Katheter bzw. ein Kathetersystem 100 auf, der einen Dreiwegehahn 1, einen mit dem ersten Anschluss 10 des Dreiwegehahns 1 verbundenen Katheterschlauch 110 und einen Druckaufnehmer 35 umfasst. Eine Steckverbindung 36 ist über ein Kabel 37 mit dem Druckaufnehmer 35 verbunden.

Weiterhin weist das Monitoring-Katheter-Set 90 eine Spül- und Blutentnahmevorrichtung 120 auf. Die Spül- und Blutentnahmevorrichtung 120 weist eine erste Schlauchleitung 125, einen Blutentnahmeport mit Silikonmembran 130, eine Spritze 135, eine Halteplatte 145 mit einer Abgleichvorrichtung 140, die eine Hydrophob-Dichtung 150 aufweist, ein Sicherheitsventil mit Spülkapillare 155, eine zweite Schlauchleitung 160, eine Tropfkammer 165 und einen Spülbeutel 170 auf. Dabei ist ein erstes Ende der ersten Schlauchleitung 125 über eine Luerkegelverbindung mit dem zweiten Anschluss 20 des Dreiwegehahns 1 verbunden. Mit der ersten Schlauchleitung 125 ist der Blutentnahmeport mit Silikonmembran 130 verbunden. Von dem Dreiwegehahn 1 aus gesehen hinter dem Blutentnahmeport 130 ist die Spritze 135 mit der ersten Schlauchleitung 125 verbunden. Das zweite Ende der ersten Schlauchleitung 125 ist mit der Abgleichvorrichtung 140 verbunden. Die Abgleichvorrichtung 140 ist auf der Halteplatte 145 befestigt, die sich auf Herzhöhe des Patienten befindet. Die Abgleichvorrichtung 140 ist mit einer Hydrophob-Dichtung 150 versehen. Mit der Abgleichvorrichtung 140 ist eine zweite Schlauchleitung 160 verbunden. Mit dieser ist das Sicherheitsventil mit Spülkapillare 155 verbunden.

An der zweiten Schlauchleitung 160 ist von der Abgleichvorrichtung 140 aus gesehen hinter dem Sicherheitsventil 155 die Tropfkammer 165 vorgesehen. Mit der Tropfkammer 165 ist der Spülbeutel 170 verbunden. Der Spülbeutel ist mit einem Druck von 300 mmHg beaufschlagt.

Mit dem Monitoring-Katheter-Set kann bei einer zweiten Schaltstellung des Dreiwegehahns 1 eine kontinuierliche Druckmessung des Pulses eines Patienten vorgenommen werden. Während der Druckmessung wird das Monitoring-Katheter-Set kontinuierlich mit einer sich in dem Spülbeutel 170 befindlichen Lösung gespült.

Der Druckaufnehmer 35 kann durch den Dreiwegehahn 1 von dem Katheterschlauch 110 ab- und angekoppelt werden und ist in der dritten Schaltstellungen des Dreiwegehahns 1 kalibrierbar. Zum Kalibrieren des Druckaufnehmers 35 wird zunächst der Dreiwegehahn 1 in seine dritte Stellung gebracht. Danach wird die zweite Schlauchleitung 160 an der Abgleichvorrichtung 140 zur Atmosphäre geöffnet. Dadurch liegt an dem Druckaufnehmer 35 Atmosphärendruck an.

In der ersten Schaltstellung des Dreiwegehahns 1 kann durch den Blutentnahmeport 130 Blut entnommen werden. In dieser Schaltstellung können dem Patienten mit der Spritze 135 Medikamente zugeführt werden.

Zur Anwendung des Monitoring-Katheter-Sets 90 wird der Sensor-Katheter 100 nach der Seldinger-Technik mit einem Führungsdraht platziert. Dabei befindet sich der Dreiwegehahn 1 in der ersten Schaltstellung. Danach wird die Spül- und Blutentnahmevorrichtung 120 an dem Sensor-Katheter 100 angeschlossen.

Dadurch, dass das Monitoring-Katheter-Set 90 kontinuierlich gespült wird, wird das Entstehen von Ablagerungen verhindert.

Dadurch, dass der Druckaufnehmer 35 nah am Patienten vorgesehen ist, und die Drucksignale nicht durch Weichkomponenten gedämpft werden, werden die Drücke im Katheter 110 besonders genau gemessen.

Dadurch, dass an dem Monitoring-Katheter-Set 90 ein Blutentnahmeport 130 und eine Spritze 135 vorgesehen sind, ist es auf einfache Weise mögliche, dem Patienten Blut abzunehmen und Medikamente zuzuführen.

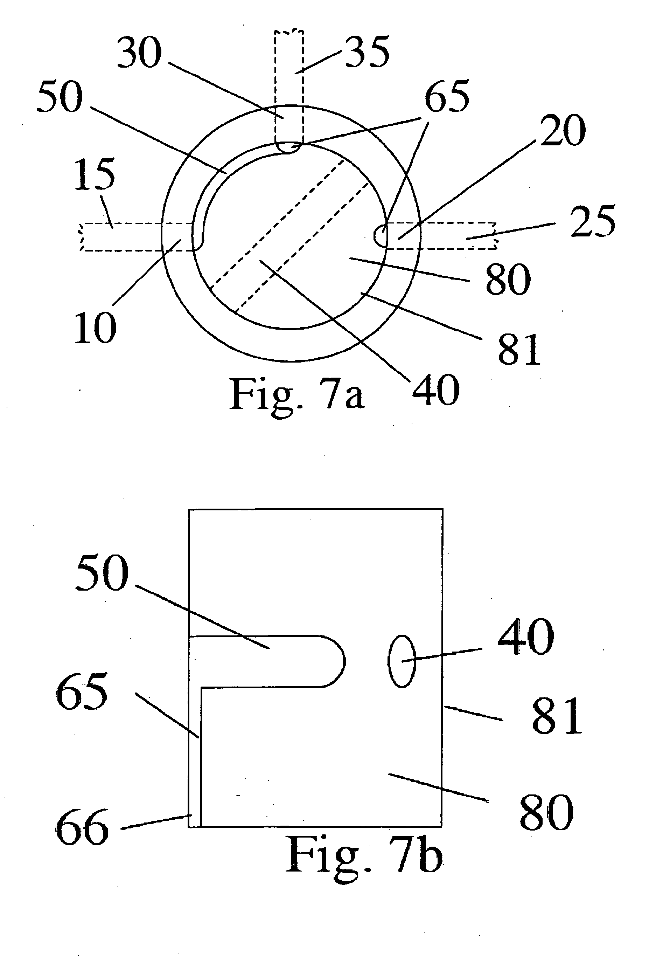

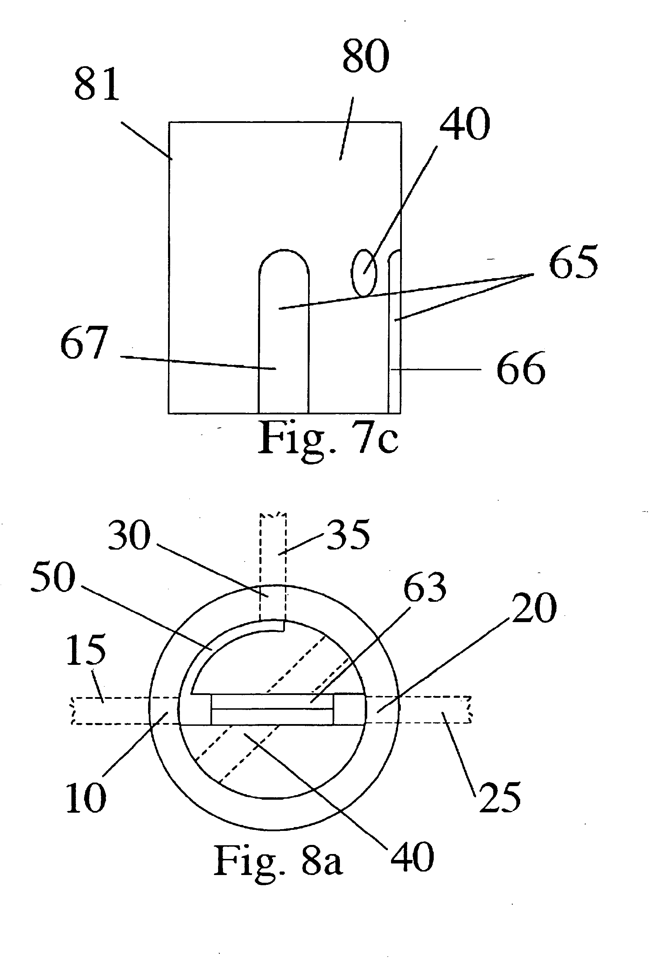

In Figur 7 ist eine Ausführungsform des Dreiwegehahns dargestellt, in der der dritte Kanal 65 als u-förmige Rille in der Außenfläche 81 der Schalteinrichtung 80 ausgebildet ist. Die beiden parallelen Bereiche 66, 67 des u-förmigen dritten Kanals 65 verlaufen auch parallel zu der Drehachse der Schalteinrichtung 80. Der erste parallele Bereich 66 des dritten Kanals 65 und der zweite Kanal 50 bilden ein "L". Der zweite Kanal 50 ist als Vertiefung in der Schalteinrichtung 80 ausgebildet und erstreckt sich in Umfangsrichtung auf der Schalteinrichtung 80.

Dadurch, dass der erste parallele Bereich 66 des dritten Kanals 65 und der zweite Kanal ein "L" bilden, ist es möglich, den Dreiwegehahn so zu spülen, dass die Spülung direkt an dem Druckaufnehmer 35 vorüber läuft.

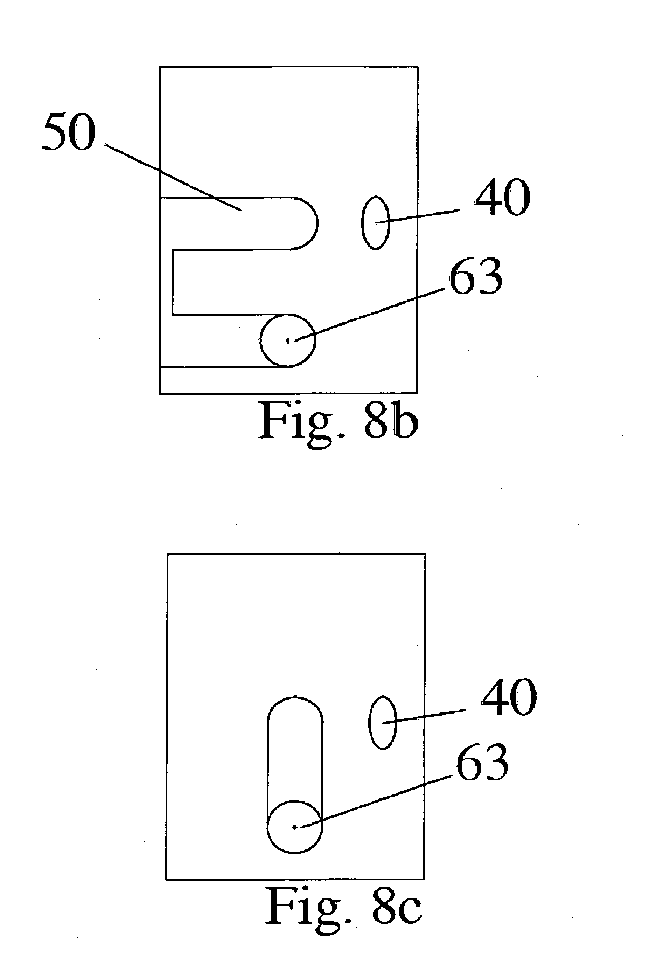

In der in Figur 8 dargestellten Ausführungsform des Dreiwegehahns ist der dritte Kanal als Glaskapillare 63 ausgeführt. Dabei ist Anschluss 20 über eine Rille senkrecht in eine andere Ebene geführt und mit einem Durchgangsloch verbunden. Dieses Durchgangsloch beinhaltet die Glaskapillare 63. Auf der gegenüberliegenden Seite ist die Glaskapillare 63 über eine C-förmige Rille mit dem Anschluss 30 verbunden.

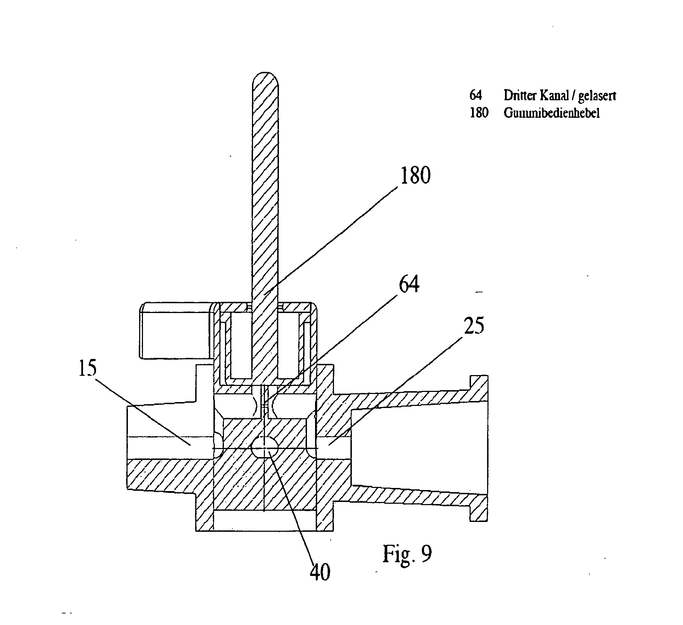

In der in Figur 9 dargestellten Ausführungsform ist die Kapillare als gelasertes Loch 64 in einem Steg realisiert. Bei dieser Ausführungsform kann durch ein Überströmen des Steges eine Schnellspülung realisiert werden. Diese Schnellspülung ist durch die Betätigung eines Dichtelements auslösbar, das von außen mit einem Gummibedienhebel 180 bedienbar ist. Durch die Verwendung des Gummibedienhebels 180 ist eine permanente Betätigung der Schnellspülung nicht möglich. Somit wird ein möglicher Anwenderfehler ausgeschlossen.

Eine kontinuierliche Spülfunktion verläuft durch den Topf des Dreiwegehahns. Dabei wird die Infusion über eine parallel zur Achse des Dreiwegehahns verlaufende Rille gelenkt. Durch die Reduktion des Querschnitts in den Rillen findet die Spülratenreduktion in den Rillen statt.

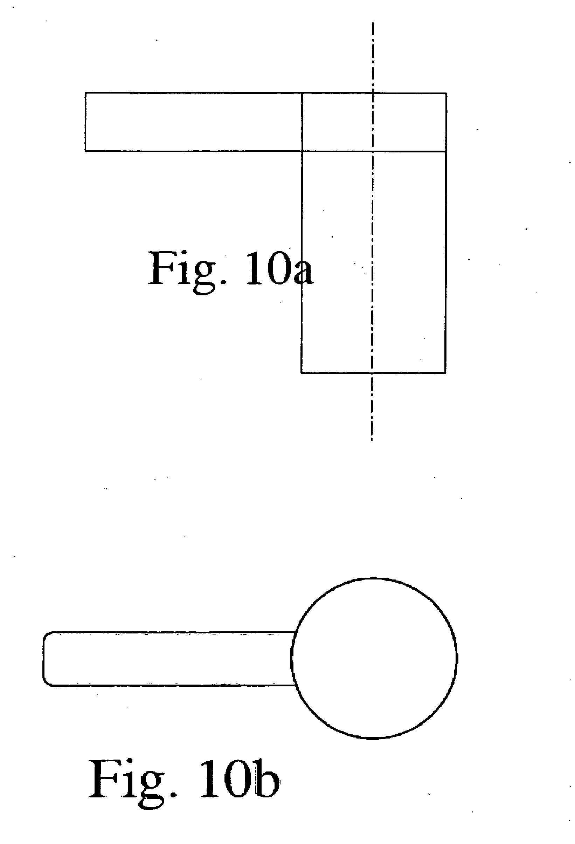

Die Bedienung des Dreiwegehahns kann auf mehrere Arten erfolgen. In den Figuren 10a und 10b ist ein Hebel zur Bedienung des Dreiwegehahns gezeigt.

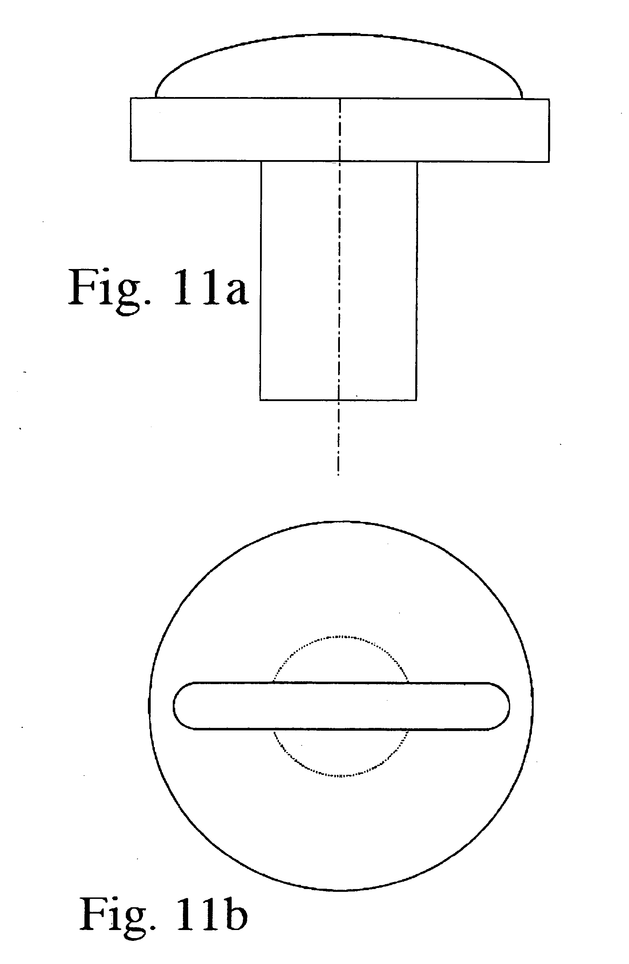

In den Figuren 11a und 11b ist ein Hebel in einer weiteren Ausführungsform zur Bedienung des Dreiwegehahns gezeigt.

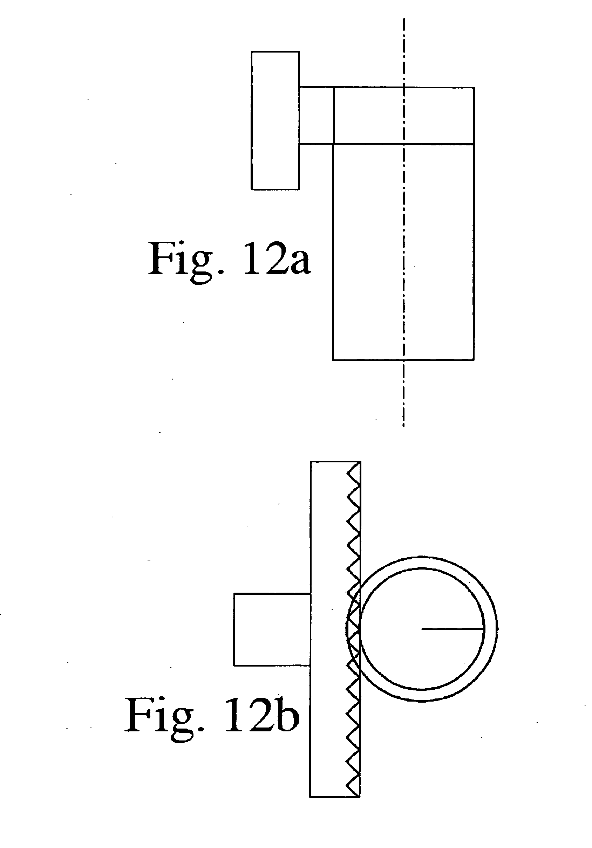

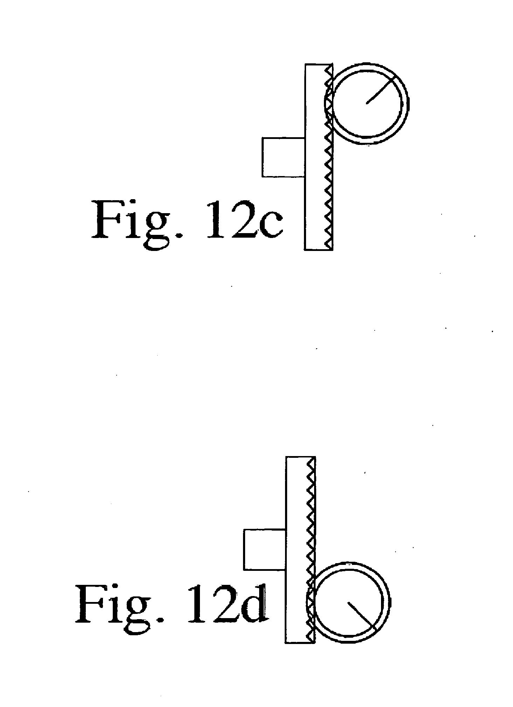

In den Figuren 12a-d ist eine Schiebemechanik dargestellt, mit der das Ventil über eine Zahnstange oder ähnliches drehbar ist.

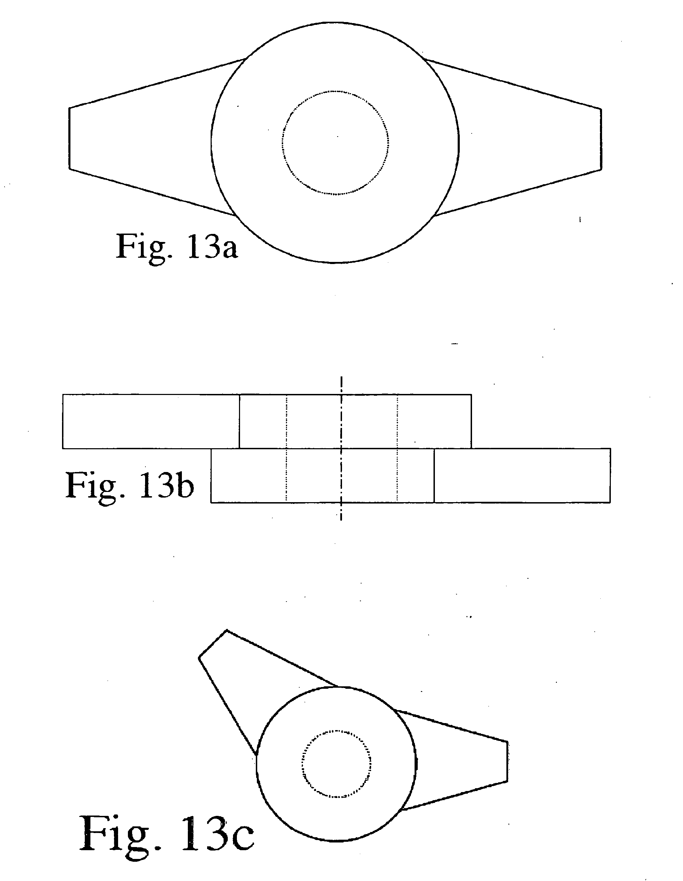

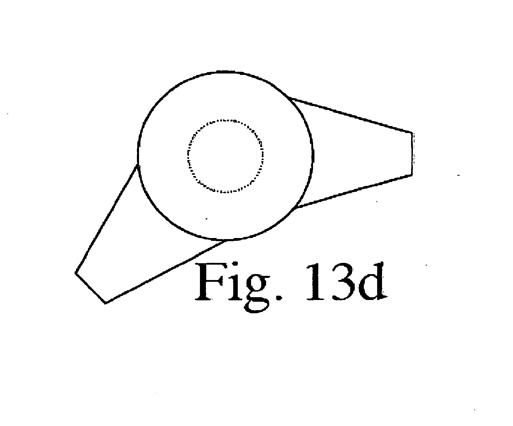

In den Figuren 13a-d ist ein Gehäuse gezeigt, das durch ein Verdrehen der Gehäusehälften gegeneinander trennbar ist.Durch das Verdrehen der Gehäusehälften werden die verschiedenen Schaltstellungen realisiert.

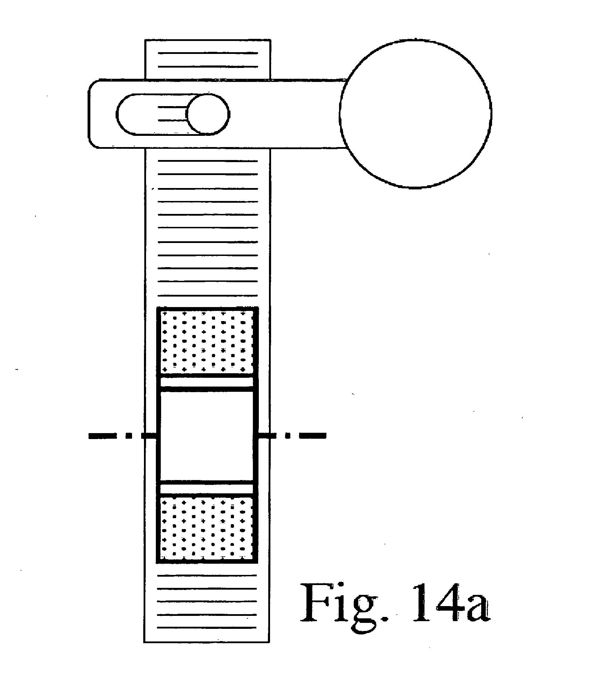

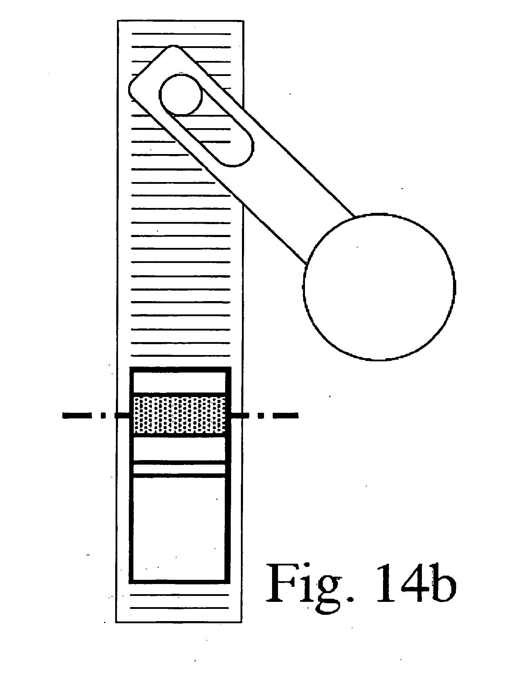

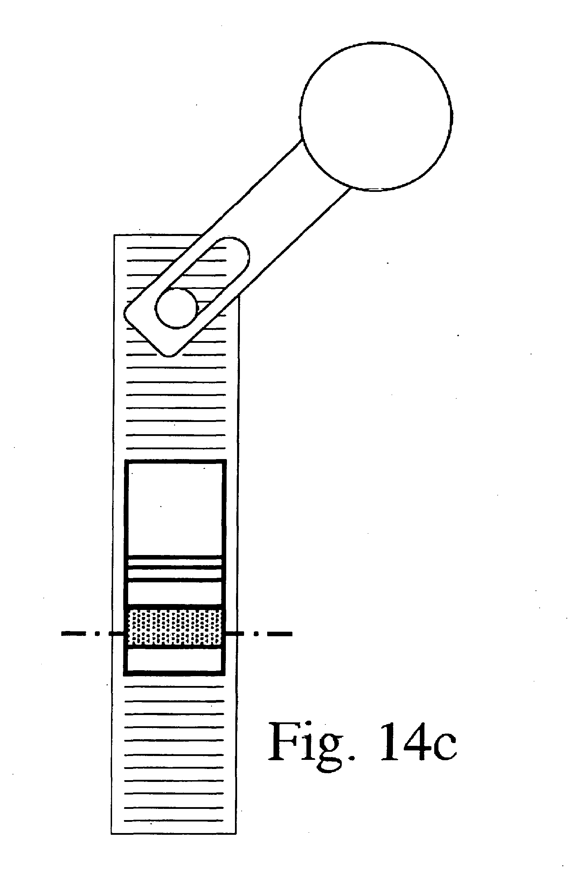

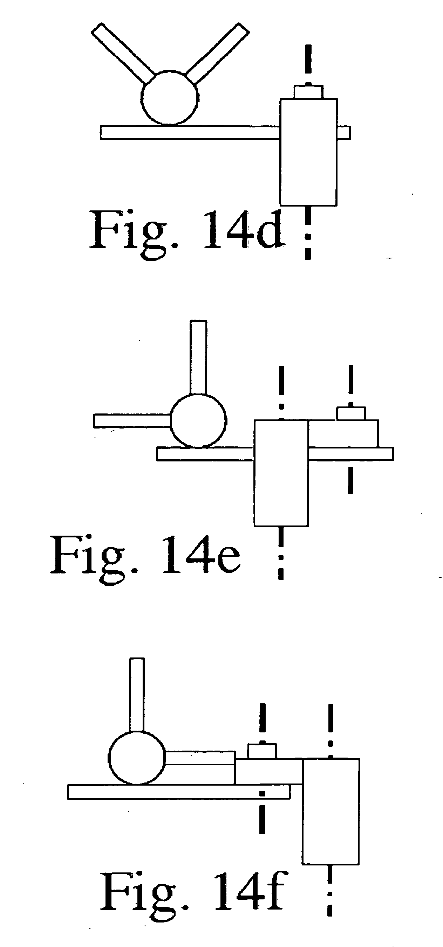

In den Figuren 14a-f ist ein Wippschalter zur Bedienung des Dreiwegehahns gezeigt. Dieser Wippschalter wandelt eine lineare in eine rotatorische Bewegung um und betätigt so das Ventil.

### Bezugszeichenliste

- 1: Dreiwegehahn
- 10: Erster Anschluss
- 15: Katheter
- 20: Zweiter Anschluss
- 25: Infusion
- 30: Dritter Anschluss
- 35: Druckaufnehmer
- 36: Steckverbindung
- 37: Kabel
- 40: Erster Kanal
- 50: Zweiter Kanal
- 51: Aufweitung
- 52: Zweiter Kanal
- 53: Öffnungen des zweiten Kanals
- 60: Dritter Kanal
- 61: Dritter Kanal
- 63: Glaskapillare
- 64: Gelasertes Loch
- 65: Dritter Kanal
- 66: Erster paralleler Bereich
- 67: Zweiter paralleler Bereich
- 70: Gehäuse
- 80: Schalteinrichtung
- 81: Außenfläche
- 90: Monitoring-Katheter-Set
- 100: Sensor Katheter
- 110: Katheterschlauch
- 120: Spül- und Blutentnahmevorrichtung
- 125: Erste Schlauchleitung
- 130: Blutentnahmeport
- 135: Spritze
- 140: Abgleichvorrichtung
- 145: Halteplatte
- 150: Hydrophob-Dichtung
- 155: Spülkapillare
- 160: Zweite Schlauchleitung
- 165: Tropfkammer
- 170: Spülbeutel

## Patentansprüche

1. Multifunktionsventil (1) mit mindestens drei Anschlüssen (10, 20, 30), wobei
ein erster Anschluss (10) mit einem Katheter, ein zweiter Anschluss (20) mit einer Infusion und ein dritter Anschluss (30) mit einem Druckaufnehmer verbindbar ist, in einer ersten Stellung der erste und der zweite Anschluss (10, 20) über einen ersten Kanal (40) miteinander verbunden sind,
in einer zweiten Stellung der erste und der dritte Anschluss (10, 30) über einen zweiten Kanal (50) miteinander verbunden sind und der zweite und der dritte Anschluss (20, 30) über einen dritten Kanal (60, 61) miteinander verbunden sind, ,
in einer dritten Stellung der zweite und der dritte Anschluss (20, 30) über den zweiten Kanal (50) miteinander verbunden sind und der erste Anschluss (10) verschlossen ist, und **dadurch gekennzeichnet, dass**
der dritte Kanal (60, 61) einen kleineren Querschnitt als der zweite Kanal (50) aufweist.

2. Multifunktionsventil (1) nach Anspruch 1, **dadurch gekennzeichnet, dass** der erste und der zweite Anschluss (10, 20) einander gegenüberliegen.

3. Multifunktionsventil (1) nach einem der vorhergehenden Ansprüche, **dadurch gekennzeichnet, dass** das Multifunktionsventil (1) ein Gehäuse (70) aufweist und der Druckaufnehmer (35) unmittelbar an dem Gehäuse (70) angebracht ist.

4. Multifunktionsventil nach einem der vorhergehenden Ansprüche, **dadurch gekennzeichnet, dass** der dritte Kanal (60, 61) eine Kapillare ist.

5. Multifunktionsventil (1) nach einem der Ansprüche 3 bis 4, **dadurch gekennzeichnet, dass** der erste Kanal (40), der zweite Kanal (50) und der dritte Kanal (60, 61) in einer Schalteinrichtung (80) angeordnet sind, die in dem Gehäuse (70) drehbar ist.

6. Multifunktionsventil (1) nach einem der vorhergehenden Ansprüche, **dadurch gekennzeichnet, dass** der erste Kanal (40) als Durchgangsloch ausgebildet ist.

7. Multifunktionsventil (1) nach einem der Ansprüche 5 oder 6, **dadurch gekennzeichnet, dass** der erste Kanal (40) quer durch die Schalteinrichtung (80) verläuft.

8. Multifunktionsventil (1) nach einem der vorhergehenden Ansprüche, **dadurch gekennzeichnet, dass** ein Richtungsvektor des ersten Kanals (40), ein Richtungsvektor des dritten Kanals (60, 61) und ein Vektor, der von einem Aufpunkt des ersten Kanals (40) zu einem Aufpunkt des dritten Kanals (60, 61) zeigt, linear unabhängig sind.

9. Multifunktionsventil (1) nach einem der vorhergehenden Ansprüche, **dadurch gekennzeichnet, dass** der erste Kanal (40) und der dritte Kanal (60) nicht mit einander verbunden sind.

10. Multifunktionsventil (1) nach einem der vorhergehenden Ansprüche, **dadurch gekennzeichnet, dass** der zweite Kanal (50) als Rille in der Schalteinrichtung (80) ausgebildet ist.

11. Multifunktionsventil (1) nach einem der vorhergehenden Ansprüche, **dadurch gekennzeichnet, dass** der zweite Kanal (50) im Wesentlichen in Umfangsrichtung der Schalteinrichtung (80) verläuft.

12. Multifunktionsventil (1) nach einem der vorhergehenden Ansprüche, **dadurch gekennzeichnet, dass** der zweite und der dritte Kanal (50, 60, 61) miteinander verbunden sind.

13. Multifunktionsventil (1) nach einem der vorhergehenden Ansprüche, **dadurch gekennzeichnet, dass** der zweite Kanal (50) in einem Endbereich eine Aufweitung aufweist und im Bereich der Aufweitung mit dem dritten Kanal (60, 61) verbunden ist.

14. Multifunktionsventil (1) nach einem der vorhergehenden Ansprüche, **dadurch gekennzeichnet, dass** der zweite Kanal (52) als Durchgangsloch ausgebildet ist.

15. Kathetersystem (100) mit einem Multifunktionsventil (1) nach einem der vorhergehenden Ansprüche, wobei das Multifunktionsventil (1) mit einem Katheterschlauch (110) verbindbar ist.

16. Kathetersystem (100) nach Anspruch 15, wobei an dem Multifunktionsventil (1) ein Druckaufnehmer (35) anschließbar ist.

17. Kathetersystem (100) nach einem der Ansprüche 15 oder 16, wobei an den Druckaufnehmer (35) eine Steckverbindung (36) anschließbar ist.

18. Vorrichtung (120), mit einem Kathetersystem (100) nach einem der Ansprüche 15 bis 17, wobei das Kathetersystem (100) mit einem Blutentnahmeport (130) verbindbar ist.

19. Vorrichtung (120) nach Anspruch 18, wobei das Kathetersystem (100) mit einem Reservoir mit beweglichem Kolben (135) verbindbar ist.

20. Vorrichtung (120) nach einem der Ansprüche 18 oder 19, wobei das Kathetersystem (100) mit einer Abgleichvorrichtung (140) verbindbar ist.

21. Vorrichtung (120) nach einem der Ansprüche 18 bis 20, wobei das Kathetersystem (100) mit einem Sicherheitsventil (155) verbindbar ist.

22. Vorrichtung (120) nach anspruch 21, wobei das Sicherheitsventil (155) mit einer Spülkapillare versehen ist.

23. Vorrichtung (120) nach einem der Ansprüche 18 bis 22, wobei das Kathetersystem (100) mit einer Tropfkammer (165) verbindbar ist.

24. Vorrichtung (120) nach einem der Ansprüche 18 bis 23, wobei das Kathetersystem (100) mit einer Spüllösungseinrichtung (170) verbindbar ist.

## Claims

1. A multifunction valve (1) comprising at least three connectors (10, 20, 30),
wherein a first connector (10) can be connected to a catheter, a second connector (20) to an infusion and a third connector (30) to a pressure transducer, wherein in a first position the first and second connectors (10, 20) are connected to each other via a first channel (40),
in a second position the first and the third connectors (10, 30) are connected to each via a second channel (50), and the second and third connectors (20, 30) are connected to each other via a third channel (60, 61),
in a third position the second and third connectors (20, 30) are connected to each other via the second channel (50), and the first connector (10) is closed, and
**characterized in that**
the third channel (60, 61) has a smaller cross-section than the second channel (50).

2. A multifunction valve (1) according to claim 1, **characterized in that** the first and second connectors (10, 20) are opposite one another.

3. A multifunction valve (1) according to any of the preceding claims, **characterized in that** the multifunction valve (1) has a housing (70) and the pressure transducer (35) is mounted directly on the housing (70).

4. A multifunction valve according to any of the preceding claims, **characterized in that** the third channel (60, 61) is a capillary.

5. A multifunction valve (1) according to any of claims 3 to 4, **characterized in that** the first channel (40), the second channel (50) and the third channel (60, 61) are arranged in a switching mechanism (80) which can be turned inside the housing (70).

6. A multifunction valve (1) according to any of the preceding claims, **characterized in that** the first channel (40) is configured as a through hole.

7. A multifunction valve (1) according to any of claims 5 or 6, **characterized in that** the first channel (40) extends transversely through the switching mechanism (80).

8. A multifunction valve (1) according to any of the preceding claims, **characterized in that** a direction vector of the first channel (40), a direction vector of the third channel (60, 61) and a vector which points from a reference point of the first channel (40) to a reference point of the third channel (60, 61) are linearly independent.

9. A multifunction valve (1) according to any of the preceding claims, **characterized in that** the first channel (40) and the third channel (60) are not connected to each other.

10. A multifunction valve (1) according to any of the preceding claims, **characterized in that** the second channel (50) is configured as a groove in the switching mechanism (80).

11. A multifunction valve (1) according to any of the preceding claims, **characterized in that** the second channel (50) extends substantially in the circumferential direction of the switching mechanism (80).

12. A multifunction valve (1) according to any of the preceding claims, **characterized in that** the second and the third channels (50, 60, 61) are connected to each other.

13. A multifunction valve (1) according to any of the preceding claims, **characterized in that** the second channel (50) has a dilation in one end portion and is connected in the region of the dilation to the third channel (60, 61).

14. A multifunction valve (1) according to any of the preceding claims, **characterized in that** the second channel (52) is configured as a through hole.

15. A catheter system (100) provided with a multifunction valve (1) according to any of the preceding claims, wherein the multifunction valve (1) can be connected to a catheter tube (110).

16. A catheter system (100) according to claim 15, wherein a pressure transducer (35) can be connected to the multifunction valve (1).

17. A catheter system (100) according to any of claims 15 or 16, in which a plug connector (36) can be connected to the pressure transducer (35).

18. An apparatus (120) provided with a catheter system (100) according to any of claims 15 to 17, wherein the catheter system (100) can be connected to a blood withdrawal port (130).

19. An apparatus (120) according to claim 18, wherein the catheter system (100) can be connected to a reservoir with a movable piston (135).

20. An apparatus (120) according to any of claims 18 or 19, wherein the catheter system (100) can be connected to a calibration device (140).

21. A device (120) according to any of claims 18 to 20, wherein the catheter system (100) can be connected to a safety valve (155).

22. A device (120) according to claim 21, wherein the catheter safety valve (155) is provided with a flushing capillary.

23. A device (120) according to any of claims 18 to 22, wherein the catheter system (100) can be connected to a drip chamber (165).

24. A device (120) according to any of claims 18 to 23, wherein the catheter system (100) can be connected to a flushing solution device (170).

## Revendications

1. Vanne (1) multifonction ayant au moins trois raccords (10, 20, 30),
dans laquelle un premier raccord (10) peut communiquer avec un cathéter, un deuxième raccord (20) avec une perfusion et un troisième raccord (30) avec un capteur de pression,
dans une première position, le premier et le deuxième raccords (10, 20) communiquent entre eux par un premier canal (40),
dans une deuxième position, le premier et le troisième raccords (10, 30) communiquent entre eux par un deuxième canal (50) et le deuxième et le troisième raccords (20, 30) communiquent entre eux par un troisième canal (60, 61),
dans une troisième position, le deuxième et le troisième raccords (20, 30) communiquent entre eux par le deuxième canal (50) et le premier raccord (10) est fermé, et
**caractérisée en ce que**
le troisième canal (60, 61) a une section transversale plus petite que le deuxième canal (50).

2. Vanne (1) multifonction suivant la revendication 1, **caractérisée en ce que** le premier et le deuxième raccords (10, 20) sont opposés l'un à l'autre.

3. Vanne (1) multifonction suivant l'une des revendications précédentes, **caractérisée en ce que** la vanne (1) multifonction a un corps (70) et le capteur (35) de pression est mis directement sur le corps (70).

4. Vanne (1) multifonction suivant l'une des revendications précédentes, **caractérisée en ce que** le troisième canal (60, 61) est un capillaire.

5. Vanne (1) multifonction suivant l'une des revendications 3 à 4, **caractérisée en ce que** le premier canal (40), le deuxième canal (50) et le troisième canal (60, 61) sont montés dans un dispositif (80) de commutation, qui peut tourner dans le corps (70).

6. Vanne (1) multifonction suivant l'une des revendications précédentes, **caractérisée en ce que** le premier canal (40) est constitué en trou traversant.

7. Vanne (1) multifonction suivant l'une des revendications 5 ou 6, **caractérisée en ce que** le premier canal (40) s'étend transversalement dans le dispositif (80) de commutation.

8. Vanne (1) multifonction suivant l'une des revendications précédentes, **caractérisée en ce qu'**un vecteur directionnel du premier canal (40), un vecteur directionnel du troisième canal (60, 61) et un vecteur, qui va d'un point donné du premier canal (40) à un point donné du troisième canal (60, 61), sont indépendamment linéaires.

9. Vanne (1) multifonction suivant l'une des revendications précédentes, **caractérisée en ce que** le premier canal (40) et le troisième canal (60) ne communiquent pas entre eux.

10. Vanne (1) multifonction suivant l'une des revendications précédentes, **caractérisée en ce que** le deuxième canal (50) est constitué sous la forme d'une rainure dans le dispositif (80) de commutation.

11. Vanne (1) multifonction suivant l'une des revendications précédentes, **caractérisée en ce que** le deuxième canal (50) s'étend sensiblement dans la direction périphérique du dispositif (80) de commutation.

12. Vanne (1) multifonction suivant l'une des revendications précédentes, **caractérisée en ce que** le deuxième et le troisième canal (50, 60, 61) communiquent entre eux.

13. Vanne (1) multifonction suivant l'une des revendications précédentes, **caractérisée en ce que** le deuxième canal (50) a un élargissement dans la partie d'extrémité et communique avec le troisième canal (60, 61) dans la partie de l'élargissement.

14. Vanne (1) multifonction suivant l'une des revendications précédentes, **caractérisée en ce que** le deuxième canal (52) est constitué en trou traversant.

15. Système (100) de cathéter ayant une vanne (1) multifonction suivant l'une des revendications précédentes, dans lequel la vanne (1) multifonction peut communiquer avec un conduit souple (110) de cathéter.

16. Système (100) de cathéter suivant la revendication 15, dans lequel un capteur (35) de pression peut être raccordé à la vanne (1) multifonction.

17. Système (100) de cathéter suivant l'une des revendications 15 ou 16, dans lequel une liaison (36) à enfichage peut être raccordée au capteur (35) de pression.

18. Dispositif (120) ayant un système (100) de cathéter suivant l'une des revendications 15 à 17, le système (100) de cathéter pouvant communiquer avec un orifice (130) de prélèvement de sang.

19. Dispositif (120) suivant la revendication 18, dans lequel le système (100) de cathéter peut communiquer avec un réservoir ayant un piston (135) mobile.

20. Dispositif (120) suivant l'une des revendications 18 ou 19, dans lequel le système (100) de cathéter peut communiquer avec un dispositif (140) d'égalisation.

21. Dispositif (120) suivant l'une des revendications 18 à 20, dans lequel le système (100) de cathéter peut communiquer avec une soupape (155) de sécurité.

22. Dispositif (120) suivant la revendication 21, dans lequel la soupape (155) de sécurité est pourvue d'un capillaire de lavage.

23. Dispositif (120) suivant l'une des revendications 18 à 22, dans lequel le système (100) de cathéter peut communiquer avec une chambre (165) de goutte-à-goutte.

24. Dispositif (120) suivant l'une des revendications 18 à 23, dans lequel le système (100) de cathéter peut communiquer avec un dispositif (170) de solution de lavage.
